# FASCICULE DE BREVET EUROPEEN

(11) **EP 3 079 713 B1**
(45) Date de publication et mention de la délivrance du brevet: **19.08.2020**
(21) Numéro de dépôt: 14818926.9
(22) Date de dépôt: 11.12.2014
(51) Int. Cl.: A61K 38/39, A61P 7/04, A61K 9/16, A61K 9/00

(54) **PREPARATIONS INJECTABLES A BASE DE COLLAGENES CAPABLES DE CONTROLER LES SAIGNEMENTS ET/OU DE SE SUBSTITUER A DES PLAQUETTES DANS LE CAS DE SYNDROMES HEMORRAGIQUES**
INJIZIERBARE ZUBEREITUNGEN AUF COLLAGENBASIS ZUR BLUTSTILLUNG UND/ODER ALS ERSATZ FÜR BLUTPLÄTTCHEN BEI HÄMORRHAGISCHEN SYNDROMEN
COLLAGEN-BASED INJECTABLE PREPARATIONS CAPABLE OF CONTROLLING BLEEDING AND/OR OF SUBSTITUTING FOR PLATELETS IN THE CASE OF HAEMORRHAGE SYNDROMES

(30) Priorité: 11.12.2013 FR 1362418
(43) Date de publication de la demande: 19.10.2016
(73) Titulaire: NVH Medicinal, 21000 Dijon (FR); Centre Hospitalier Universitaire De Dijon, 21000 Dijon (FR); Vandroux, David, 21000 Dijon (FR)
(72) Inventeur: VANDROUX, David, F-21000 Dijon (FR); DUMONT, Laure, F-21000 Dijon (FR); DE MAISTRE, Emmanuel, F-21121 Fontaine-les-Dijon (FR)
(74) Mandataire: Regimbeau
(86) Numéro de dépôt international: PCT/EP2014/077385
(87) Numéro de publication internationale: WO 2015/086748

(56) Documents cités:
- WO-A1-2014/060568
- WO-A2-98/57678
- FR-A1- 2 936 247
- US-A1- 2006 100 138
- US-A1- 2009 299 034
- GEUTJES P J ET AL: "Preparation and characterization of injectable fibrillar type i collagen and evaluation for pseudoaneurysm treatment in a pig model", JOURNAL OF VASCULAR SURGERY 2010 MOSBY INC. USA, vol. 52027442941, no. 5, novembre 2010 (2010-11), pages 1330-1338, XP027442941, ISSN: 0741-5214
- LISMAN TON ET AL: "A single high-affinity binding site for von Willebrand factor in collagen III, identified using synthetic triple-helical peptides", BLOOD, AMERICAN SOCIETY OF HEMATOLOGY, US, vol. 108, no. 12, 1 décembre 2006 (2006-12-01), pages 3753-3756, XP002425786, ISSN: 0006-4971, DOI: 10.1182/BLOOD-2006-03-011965
- ANDREW B HERR ET AL: "Structural Insights into the Interactions between Platelet Receptors and Fibrillar Collagen", JOURNAL OF BIOLOGICAL CHEMISTRY, AMERICAN SOCIETY FOR BIOCHEMISTRY AND MOLECULAR BIOLOGY, US, vol. 284, no. 30, 24 juillet 2009 (2009-07-24), pages 19781-19785, XP002693781, ISSN: 0021-9258, DOI: 10.1074/JBC.R109.013219 [extrait le 2009-04-28]

## Description

### DOMAINE DE L'INVENTION

L'invention porte sur une préparation injectable à base de collagènes pour son utilisation dans le traitement des hémorragies. La préparation injectable comprend des protéines et peptides dérivés du collagène capables, à eux seuls, d'induire à la fois l'adhésion et l'activation des plaquettes, voire même leur agrégation. La présente description divulgue également sur l'injection par voie systémique/générale, en urgence, de protéines et peptides dérivés du collagène chez des patients traités par des antiplaquettaires en situation d'urgence (hémorragie, intervention chirurgicale).

### ARRIERE PLAN DE L'INVENTION

L'hémorragie se définit comme un écoulement de sang en dehors de la circulation sanguine normale. Elle peut consister en un simple saignement ou devenir massive dans certaines situations, notamment traumatiques, et aboutir à un état de choc hémorragique associé à une chute de la tension artérielle.
Différents contextes cliniques conduisent à un risque hémorragique tels que la chirurgie, un traumatisme, un traitement, une thrombopénie ou un déficit constitutionnel ou fonctionnel (comme l'hémophilie) (Spahn D et coll. Critical Care 2013, 17:R76). Dans les hémorragies sévères, une coagulopathie est observée. La coagulopathie correspond à une consommation des facteurs plaquettaires et de la coagulation. Sa prise en charge est une urgence vitale qui conditionne fortement l'issue de cette hémorragie. Différentes stratégies de prise en charge sont actuellement suivies.
Une approche consiste à utiliser un support transfusionnel à base de plasma frais congelé (PFC), de concentré globulaire ou de concentré plaquettaire. Cependant, le succès de l'approche à base de plaquettes est limité par plusieurs facteurs parmi lesquels l'accessibilité aux plaquettes, leur durée de vie réduite (5 jours en moyenne), les inconvénients liés aux conditions de conservation, au risque de contamination de ce type d'échantillons, leur coût et le risque d'inefficacité voire d'intolérance par alloimmunisation.
Une autre approche est basée sur l'utilisation de concentrés en facteurs de la coagulation tels que les concentrés en fibrinogène et les concentrés en complexe prothrombine (facteurs II, VII, IX et X). Des études ont également suggéré l'utilisation du facteur VIIa (NovoSeven®).

Cependant cette dernière utilisation n'est pas recommandée du fait du taux élevé de thrombose induite.
Enfin, une autre approche consiste à utiliser, dans des situations d'urgence, de l'acide tranexamique qui est un antagoniste puissant de l'activation du plasminogène et donc se comporte comme un anti-fibrinolytique (Fries D. Transfusion 2013;53:91S-95S).
Aucune des approches proposées à ce jour ne permet d'induire l'activation et l'agrégation des plaquettes au cours d'un épisode hémorragique, alors que celles-ci correspondent à des étapes clé dans l'arrêt des saignements. En effet, lors d'une atteinte vasculaire conduisant à un saignement, une suite d'étapes, regroupée sous le terme d'hémostase primaire, se met en place. Elle implique les plaquettes, cellules anucléées de forme discoïdale biconvexe et de diamètre approximatif de 2 à 5 µm. Différents acteurs participent à ce processus qui conduit à la formation d'un clou hémostatique. La première phase dite d'adhésion permet le recrutement au niveau du site lésé de plaquettes circulantes. Cette étape fait intervenir le facteur Willebrand, le collagène et des récepteurs plaquettaires dits récepteurs au collagène (GpVI et α2β1). La deuxième phase dite d'activation plaquettaire se traduit notamment par la libération de facteurs pro-agrégants et l'expression en surface des plaquettes de protéines telles que la P-sélectine. Cette phase est suivie de la phase dite d'agrégation faisant notamment intervenir le fibrinogène et un récepteur plaquettaire GpIIb/IIIa et l'exposition membranaire de phospholipides procoagulants permettant la fixation sur site des facteurs de la coagulation et *in fine* la formation du caillot fibrino-plaquettaire et l'arrêt du saignement.
Il est maintenant bien admis que l'activation des plaquettes au cours du processus d'hémostase conduit à l'apparition de deux populations de plaquettes activées dites pro-agrégantes et pro-coagulantes. Cette dernière se caractérise par l'expression en surface de la phosphatidylsérine, à l'origine de la génération de thrombine. On les qualifie alors de plaquettes superactivées. C'est ainsi que l'on parle de potentiel de superactivation plaquettaire pour décrire la capacité de chaque individu à générer cette population de plaquettes en réponse à des agonistes tels que la thrombine et le collagène. De récents travaux suggèrent que toute stratégie thérapeutique visant à induire l'apparition de cette population de plaquettes activées présente un fort potentiel pour le traitement de situations hémorragiques (Mazepa M et coll. ATVB 2013, 33(8) :1747-52). Par ailleurs leur intérêt est renforcé par des données confirmant que l'effet hémostatique des plaquettes transfusées est dépendant de cette population. Cependant, les approches proposées à ce jour ne sont pas basées sur l'injection dans la circulation sanguine d'activateurs physiologiques ou naturels des plaquettes tels que le collagène ou les protéines et peptides dérivés du collagène revendiqués dans la présente invention.
Le rôle central des plaquettes dans l'arrêt des saignements est à l'origine de travaux récents visant à développer des produits dérivés ou mimant les plaquettes (plaquettes synthétiques).

Parmi les approches proposées, sont distinguées celles basées sur des produits dérivés de cellules, telles que les thromboérythrocytes et les thrombosomes, ou celles utilisant des particules de taille micrométrique recouvertes de peptides dérivés de protéines impliquées dans la cascade d'activation des plaquettes, telles que les synthocytes ou Fibrocaps™ à base de peptides dérivés du fibrinogène (peptides RGD ou dodécapeptide H12), ou celles recouvertes de peptides de liaison au facteur Willebrand, au collagène et à GpIIb/IIIa (Lashof-Sullivan M et coll. Nanoscale 2013, 5, 10719-10728). Un effet hémostatique a été décrit pour ces particules recouvertes de peptides de liaison au facteur Willebrand, au collagène et à GpIIb/IIIa laissant entrevoir une application dans le contexte hémorragique. Cependant, cette approche pose un problème majeur d'industrialisation pour ce type de produit qui combine plusieurs peptides au niveau d'une seule particule avec, à la clé, la nécessité de maitriser et de qualifier des paramètres tels que le taux de greffage et le ratio pour chacun des peptides ainsi que la stabilité du produit une fois injecté, sans compter le fait de devoir injecter à ce jour des doses très importantes (de l'ordre de plusieurs dizaines de mg par kg) (Modery-Pawlowski C et coll. Biomaterials 2013 :516-541).

Un besoin existe donc pour la mise à disposition de nouveaux moyens de traitement des hémorragies injectables dans la circulation sanguine, qui de manière avantageuse soient facilement industrialisables et puissent être employés à de faibles concentrations.

Avec le vieillissement de la population, les médecins sont de plus en plus souvent confrontés à des patients traités par des anticoagulants et des antiplaquettaires. Alors que la stratégie d'arrêt et de relais de ces traitements est bien définie pour des actes programmés (arrêt entre 5 et 7 jours pour les nouveaux antiplaquettaires et 5 jours pour les nouveaux anticoagulants), ces derniers compliquent fortement la prise en charge de patients, en urgence, lors de situations hémorragiques (chirurgie ou traumatisme, crânien notamment) *(*Bonhomme F et coll. Eur J Intern Med. 2014 Mar;25(3):213-20*).* Différentes stratégies de réversion en urgence ont été proposées, mais pour les nouveaux antiplaquettaires, elles se limitent principalement à la transfusion de plaquettes (Beynon C et coll. Crit Care. 2012 Jul 26;16(4):228*).* Il existe un besoin de nouveaux produits hémostatiques injectables capables de permettre la récupération d'une fonction plaquettaire lors des épisodes hémorragiques et ce, indépendamment du mode d'action de ces nouveaux antiplaquettaires. Le fait que la voie d'activation des plaquettes dépendante du collagène soit la plus physiologique et ne soit pas, à ce jour, moduler par un traitement antiplaquettaire présent sur le marché, renforce l'intérêt d'une administration par voie systémique de protéines ou peptides dérivés du collagène dans ces situations. Il est bien démontré que la liaison de GPVI au collagène ou les protéines ou peptides dérivés du collagène de la présente invention entrainent une intense signalisation intra-plaquettaire.

Dans un contexte logistiquement contraint, tel que les théâtres d'opérations extérieures pour les militaires, la prise en charge d'un tableau hémorragique s'inscrit dans le concept de « *damage control resuscitation* » et passe notamment par une réanimation transfusionnelle (concentrés en globule rouge, plasma et plaquettes). Cependant, cette situation se distingue par une difficulté d'accès à ces produits sanguins, notamment aux concentrés plaquettaires, nécessitant une prise en charge adaptée, regroupée sous le terme de « *remote damage control resuscitation* », avec comme principale nouveauté, l'utilisation de sang total (Jenkins DH et coll. Shock. 2014 May;41 Suppl 1:3-12)*.* Cette stratégie, bien qu'associée à un risque de contamination, est prometteuse mais reste limitée à ce jour au domaine militaire *(*Murdock AD et coll. Shock. 2014 May;41 Suppl 1:62-9*)*.

La présente invention porte donc sur l'intérêt de l'administration, par voie intraveineuse, de protéines ou peptides dérivés du collagène activateurs des plaquettes sanguines, comme nouvel hémostatique injectable dans trois situations impliquant une hémorragie:
- lors d'un épisode de saignement massif, notamment non compressif, nécessitant de pouvoir activer les plaquettes sanguines dans la circulation générale,
- comme adjuvant lors d'une réanimation transfusionnelle à distance d'un centre de soin, en complément d'une approche basée sur la transfusion en particulier de sang total ou de concentrés plaquettaires,
- comme agent de réversion des nouveaux antiplaquettaires (e.g. prasugrel, ticagrelor), produits actuellement commercialisés sans antidote, lors d'une chirurgie ou d'une hémorragie notamment intracrânienne.

### BREVE DESCRIPTION DE L'INVENTION

La présente invention porte sur une préparation injectable pour son utilisation dans le traitement des hémorragies comprenant :
- des particules, , lesdites particules comprenant des protéines ou peptides induisant l'adhésion et l'activation des plaquettes, voire même leur agrégation ; et
- au moins un véhicule ou excipient pharmaceutiquement acceptable.

### BREVE DESCRIPTION DES FIGURES

La figure 1 présente la mesure de la taille des particules par DLS après concentration d'un facteur 50 d'un extrait protéique du polypeptide de SEQ n°1.
La figure 2 présente un résultat obtenu par ELISA de la liaison du facteur Willebrand purifié (Willfactin, LFB) utilisé à 2 UI/dL final aux polypeptides de SEQ n°1 sous forme de particules de différentes tailles, utilisés à 2 µg/mL final et au collagène de type I (Collagène SIRCOL, contrôle positif) utilisé à 10 µg/mL.
La figure 3A représente un profil type de l'expression de la P-sélectine en surface de plaquettes obtenu en cytométrie en flux pour le polypeptide de SEQ n°1 et du collagène de type I (Horm).
La figure 3B représente les intensités moyennes de fluorescence obtenues pour différents agonistes.
La figure 4 présente un résultat d'agrégation obtenu avec le polypeptide de SEQ n°1 sous forme de particules.
La figure 5 présente la structure macromoléculaire observée par différentes techniques de microscopie du polypeptide de SEQ n°1 sous forme de particules en comparaison avec du collagène de type I (Horm) sous forme fibrillaire.
La figure 6 représente les intensités moyennes de fluorescence obtenues en cytométrie en flux de l'expression de la P-sélectine en surface de plaquettes prélevées après injection chez la souris du polypeptide de SEQ n°1 et du collagène de type I (Horm).
La figure 7 présente les résultats de l'injection par voie intraveineuse chez la souris, en présence d'épinéphrine, du collagène de type I (Horm) sous forme fibrillaire et le polypeptide de SEQ n°1 sous forme de particules dans un modèle d'embolie pulmonaire induit chez la souris. La figure 7A présente les résultats obtenus sur la mortalité des souris après injection et la figure 7B présente la biodistribution de macroagrégats d'albumines radiomarquées au technétium injectée au minimum 5 minutes après l'administration des différents collagènes testés.
La figure 8 présente les résultats de l'injection chez la souris du polypeptide de SEQ n°1 dans un modèle de saignement induit à la queue chez la souris.

### DESCRIPTION DES SEQUENCES

SEQ IN N°1 : polypeptide codant pour une protéine recombinante capable d'induire l'adhésion et l'activation des plaquettes.

### DESCRIPTION DETAILLEE DE L'INVENTION

La présente invention porte sur une préparation injectable pour son utilisation dans le traitement des hémorragies comprenant :
- des particules, lesdites particules comprenant des protéines ou peptides induisant l'adhésion et l'activation des plaquettes ; et
- au moins un excipient ou véhicule pharmaceutiquement acceptable.

Les préparations injectables selon la présente invention permettent de stimuler l'activité des plaquettes. Dans certains modes de réalisation, les particules comprennent des protéines ou peptides induisant l'adhésion et l'activation des plaquettes et également l'agrégation des plaquettes une fois injectée dans la circulation sanguine. Ces dernières peuvent également être utilisées comme adjuvant pour pré-activer des plaquettes lors de la transfusion de concentrés plaquettaires ou de sang total.

### Protéines ou peptides induisant l'adhésion, l'activation voire l'agrégation des plaquettes

Les protéines ou peptides entrant dans la formulation des préparations de la présente invention possèdent la capacité d'induire à la fois l'adhésion et l'activation des plaquettes, voire également l'agrégation des plaquettes. Il doit être compris que ces deux ou trois activités sont portées par une seule et même protéine ou peptide. Ces protéines ou peptides sont sous forme de particules.

Avantageusement les fibrilles ici décrites ont une longueur inférieure à 9µm, 8µm, 7µm, 6µm, 5µm, 4µm, 3µm, 2µm, 1µm ou 0,5µm. Plus avantageusement les fibrilles ont une longueur comprise entre 0,5 et 9µm, entre 1 et 5µm ou encore entre 2 et 4µm.

Ces protéines ou peptides sont capables de lier voire d'activer le facteur Willebrand, responsable de l'adhésion des plaquettes et d'induire l'expression de surface de la P-sélectine voire de la phosphatidylsérine, marqueurs d'activation des plaquettes. Dans certains modes de réalisation, les protéines ou peptides sont capables d'induire *in vitro* l'agrégation d'un plasma riche en plaquettes ou en sang total.

Dans certains modes de réalisation, les protéines ou peptides peuvent stimuler les plaquettes et induire l'arrêt d'un saignement sans nécessairement être capables d'induire l'activation de la coagulation, dépendante de l'expression en surface des plaquettes de la phosphatidylsérine et la formation d'une surface dite procoagulante et la génération de thrombine qui en découle. Ceci permet de limiter le risque de thrombose induite après une injection systémique de la préparation. L'activation de cette sous population de plaquettes activées, dites plaquettes procoagulantes, peut néanmoins se produire mais en deçà du niveau nécessaire à la génération de thrombine détectable par des tests classiques.

Une famille de protéines, les collagènes, possède la capacité d'induire les trois activités ci-dessus mentionnées grâce à la présence dans la séquence de certains collagènes, principalement les collagènes de type I et III, de motifs peptidiques capables de se lier au facteur Willebrand circulant ainsi qu'à deux récepteurs plaquettaires, GpVI et α2β1.

L'ensemble concourt à l'adhésion des plaquettes, à leur activation puis à leur agrégation. Ainsi, les protéines entrant dans la formulation des préparations de la présente invention peuvent être choisies parmi les collagènes, en particulier les collagènes de type I et III.

A ce jour, il n'a cependant pas été proposé d'utiliser du collagène pour traiter les hémorragies. Ceci peut être expliqué par le fait que parmi les collagènes natifs, seuls ceux sous forme fibrillaire sont décrits comme capables d'induire l'adhésion, l'activation et l'agrégation des plaquettes *(*Clementson K. Thrombosis Research 2012 129 220-224*).* Cependant, cette forme fibrillaire, avec des fibres pouvant atteindre plusieurs micromètres de long, n'est pas compatible avec une injection dans la circulation sanguine. Les formes particulaires de la présente invention sont compatibles avec une injection dans la circulation sanguine.

Des formes particulaires du collagène, telles que les microsphères, les microflorettes, les dendrimères peuvent être obtenues par des méthodes telles que décrit par Pires M et Chmielewski J. J. Am. Chem. Soc. 2009 131,2706-2712*;* Przybyla D et coll. J. Am. Chem. Soc. 2013, 135,3418-3422; Kojima C et coll. J. Am. Chem. Soc. 2009 131, 30652-6053*;* Slatter D et coll. Peptides 2012 36, 86-93*;* Kar et coll. Jounal of Biological Chemistry 2006 vol 281, n°44 33283-33290 . Pour obtenir ces formes particulaires, différentes approches ont été utilisées, basées notamment sur l'utilisation de métaux, de sels, sur l'ajout de cystéine (ponts disulfures) ou d'acides aminés aromatiques dans la séquence peptidique ou encore sur le ratio entre acides aminés de nature différente (polaires, apolaires, acides, basiques).

Outre les collagènes, les protéines entrant dans la formulation des préparations de la présente invention peuvent être des protéines recombinantes telles que décrites dans la demande de brevet internationale WO 2010/034718. Ces protéines recombinantes dérivées du collagène sont capables d'induire l'agrégation des plaquettes de façon équivalente à du collagène natif. D'autres protéines utiles dans la présente invention et dérivées du collagène sont telles que décrites dans la demande de brevet européen EP 2013/071816. Ces protéines sont capables de se lier au facteur Willebrand, liaison impliquée dans l'adhésion initiale des plaquettes au niveau d'un site vasculaire lésé.

Ainsi, les protéines entrant dans la formulation des préparations de la présente invention peuvent être choisies parmi les collagènes natifs, tels que les collagènes de type I ou III, ou les protéines recombinantes dont la séquence inclut au moins un polypeptide choisi parmi :
- le polypeptide de la séquence ID n°1 ;
- un polypeptide ayant des séquences formées de la répétition de triplets GXY où G désigne la glycine et X et Y n'importe quels acides aminés répétés n fois avec n supérieur ou égal à 2 ;
- le polypeptide ayant la séquence de la position 25 à 184 de la séquence ID n°1 ; ou
- un polypeptide présentant au moins 70% d'identité sur toute sa longueur avec le polypeptide de la séquence ID n°1 ou avec le polypeptide ayant la séquence de la position 25 à 184 de la séquence ID n°1.

En outre, peuvent être considérés comme des dérivés de collagène, des peptides ou polypeptides, naturel ou synthétique, possédant dans leur séquence une glycine tous les 3 acides aminés *(*An B et coll. Frontiers in chemistry. 2014 June;2 article 40*).* Cette structure primaire de la chaîne induit une conformation de la chaine peptidique de la même manière que le collagène en présence d'acides aminés (exemple des cystéines) ou de séquences peptidiques permettant la forme de structures multimériques (exemples des domaines de trimérisation des protéines MBL [Mannose Binding Lectin]).

Le peptide de la position 1 à la position 24 de la SEQ ID No. 1 correspond au peptide signal permettant la sécrétion de la protéine recombinante par une cellule hôte. Ce peptide signal pourra être absent ou remplacé par un autre peptide signal selon des techniques bien connues de l'homme du métier. L'homme du métier saura choisir le peptide signal homologue ou hétérologue approprié pour l'expression et la sécrétion des polypeptides dans différents systèmes d'expression procaryotes ou eucaryotes. De préférence, les polypeptides sont produits dans des cellules ou des organismes eucaryotes et en particulier dans des cellules de mammifères. Dans un mode de réalisation particulier de l'invention, les polypeptides comprennent un peptide signal permettant leur sécrétion dans le milieu extracellulaire. Dans une autre mode de réalisation, le polypeptide mature est obtenu après clivage du peptide signal.

Dans des certains modes de réalisation, la séquence des protéines recombinantes inclut au moins un polypeptide comprenant les motifs peptidiques suivants :
- GX₁X₂GER dans lequel X₁ et X₂ représentent indépendamment un acide aminé choisi parmi A, R, N, D, Q, E, G, H, I, K, M, F, P, S, T, W, Y, V et O ;
- (GPX₃)ₙ avec n compris entre 4 et 10 et X₃ représente P ou O ; et
- GPRGQX₄GVMGFX₅ où X₄ et X₅ représentent indépendamment P ou O.
P est la proline et O est l'hydroxyproline.

Dans des certains modes de réalisation, la séquence des protéines recombinantes inclut au moins un polypeptide comprenant les motifs peptidiques suivants :
- GAPGER,
- KPGEPGPK,
- (GPP)ₙ avec n compris entre 4 et 10,
- RGD.

Les protéines recombinantes comprennent ainsi :
(a) des séquences peptidiques présentant au minimum une répétition de 4 triplets GPO ;
(b) des séquences peptidiques ayant une activité de liaison aux différents récepteurs plaquettaires et présentes dans la séquence native des collagènes ; et
(c) des séquences de liaison formés de la répétition de triplets GXY entre les séquences (a) et (b) dans lesquels G désigne la glycine et X et Y désignent n'importe quel acide aminé.

Les protéines recombinantes entrant dans la formulation des préparations de la présente invention peuvent présenter une séquence incluant au moins un polypeptide présentant au moins 70% d'identité sur toute sa longueur avec le polypeptide de la séquence ID n°1 ou avec le polypeptide ayant la séquence de la position 25 à 184 de la séquence ID n°1. Dans certains modes de réalisation, les polypeptides présentent au moins 70%, 80%, 90%, 95%, 98% et préférentiellement au moins 99% d'identité avec le polypeptide de la SEQ ID No. 1. Le pourcentage d'identité désigne le pourcentage d'acides aminés identiques (acides aminés invariants ou inchangés entre deux séquences). Ces polypeptides peuvent présenter une délétion, une addition ou une substitution d'au moins un acide aminé par rapport au polypeptide de la SEQ ID No. 1.

Les méthodes de mesure du pourcentage d'identité entre polypeptides sont connues de l'homme du métier. Vector NTi 9.1.0, programme d'alignement AlignX (Clustal W algorithm) (Invitrogen INFORMAX, http ://www.invitrogen.com) peut être utilisé. De préférence, les paramètres par défaut sont utilisés.

Les protéines recombinantes peuvent être produites par des bactéries et des cellules de mammifères par des méthodes bien connues de l'homme du métier, en particulier telles que décrites dans WO 2010/034718 et EP 2013/071816.

Dans certains modes de réalisation, les protéines sont également capables de se lier au collagène présent au niveau des sites vasculaires lésés, notamment via la présence dans leur séquence d'un motif GPO répété n fois avec n = 3 à 10.

Dans certains modes de réalisations, les protéines peuvent être PEGylées ou PASylées (addition n fois des acides aminés proline, alanine et sérine).

Les peptides entrant dans la formulation des préparations de la présente invention peuvent être tels que décrits ci-dessus. Ainsi, les peptides peuvent être choisis parmi :
- le polypeptide de la séquence ID n°1 ;
- un polypeptide ayant des séquences formées de la répétition de triplets GXY où G désigne la glycine et X et Y n'importe quels acides aminés répétés n fois avec n supérieur ou égal à 2 ;
- le polypeptide ayant la séquence de la position 25 à 184 de la séquence ID n°1 ; ou
- un polypeptide présentant au moins 70% d'identité sur toute sa longueur avec le polypeptide de la séquence ID n°1 ou avec le polypeptide ayant la séquence de la position 25 à 184 de la séquence ID n°1.

Les polypeptides sont isolés ou purifiés de leur environnement naturel. Les polypeptides peuvent être préparés au moyen de différents procédés. Ces procédés sont notamment la production de polypeptides recombinants par des cellules hôtes appropriées et leur purification ultérieure, la production par synthèse chimique ou, enfin, une combinaison de ces différentes approches. Ces différents procédés de production sont bien connus de l'homme du métier. De préférence, les polypeptides sont produits par des cellules procaryotes ou eucaryotes recombinantes. Les polypeptides peuvent ainsi être produits dans des bactéries ou dans des cellules de mammifères.

Dans certains modes de réalisation de l'invention, les polypeptides sont glycosylés. Le polypeptide de la SEQ ID No. 1 possède notamment des sites de O-glycosylation présentant sur les acides aminés lysines présents en position 102 et 141 (en position 3 d'un triplet GXY). Dans un mode de réalisation préféré, le résidu asparagine en position 93 du polypeptide de la SEQ ID No. 1 est glycosylé.

### Protéines sous forme fibrillaire

Les protéines entrant dans la formulation des préparations injectables faisant objet de la présente description peuvent être structurées sous forme de fibrilles présentant une longueur inférieure à 10 µm. Les formes fibrillaires comprenant des fibres présentant une longueur supérieure à 10 µm et pouvant atteindre plusieurs micromètres de long ne sont pas compatibles avec une injection dans la circulation sanguine.

La longueur d'une fibrille peut être mesurée par des méthodes bien connues de l'homme du métier, en particulier par microscopie comme la microscopie à force atomique (AFM).

### Particules

Les particules entrant dans la formulation des préparations de la présente invention peuvent comprendre, ou consister en, des protéines ou peptides induisant l'adhésion et l'activation des plaquettes, voire leur agrégation. Les protéines et peptides sont tels que décrits ci-dessus.

Dans certains modes de réalisation, faisant objet de la présente description les particules peuvent être obtenues par adhésion des protéines ou peptides sur un support. Ainsi, les particules entrant dans la formulation des préparations de la présente invention comprennent des supports sur lesquels sont adhéré(e)s des protéines ou peptides induisant l'adhésion, l'activation voire l'agrégation des plaquettes.

Les supports peuvent être des microparticules organiques ou inorganiques. Des exemples de microparticules incluent l'albumine, des lipides, des métaux tels que l'or, le titane, le fer, l'argent ou leurs oxydes, du graphène, de la silice ou des polymères.

L'adhésion des protéines ou peptides sur les supports peut se faire par liaison covalente via des fonctions présentes en surface de la particule ou via un agent de liaison bifonctionnel ou un polymère bifonctionnel. L'agent de liaison bifonctionnel ou le polymère bifonctionnel permet de fonctionnaliser les microparticules. Il présente à l'une de ses extrémités un groupement fonctionnel lui permettant de se greffer de manière covalente en surface des microparticules, et à l'autre extrémité, un groupement fonctionnel lui permettant de se greffer de manière covalente aux protéines ou peptides.

De préférence, le polymère bifonctionnel est un polymère hétérobifonctionnel. La présence de deux groupements fonctionnels différents permet de limiter les couplages non désirés, tels que le greffage des deux extrémités du polymère sur les microparticules. De préférence, l'affinité entre les groupements fonctionnels terminaux du polymère est également faible afin de limiter l'auto-condensation du polymère par interaction entre ces deux extrémités.

Différents types de polymères bifonctionnels peuvent être utilisés pour fonctionnaliser les microparticules. Ainsi, les polymères bifonctionnels peuvent être choisis parmi les polyéthylène glycols (PEG), les poly(acide lactique-coglycolique) (PLGA), les poly(caprolactone) (PLCL), les poly(acide lactique) (PLA), les polymères de glycolide (PGA), les chitosan et dextran, *etc.* De préférence, le polymère est un polyéthylène glycol bifonctionnel, de préférence hétérobifonctionnel. La masse moléculaire du polymère varie généralement de 500 Da à 10 000 Da, de préférence de 2000 à 8000 Da, de préférence aux alentours de 5000 Da.La masse moléculaire du polymère est typiquement choisie de manière à ce que lorsqu'une protéine ou peptide est couplé(e)aux microparticules par l'intermédiaire du polymère bifonctionnel, la chaîne du polymère atteigne une longueur suffisante pour que les propriétés de la protéine ou du peptide ne soient pas altérées par la présence de la microparticule.

Dans certains modes de réalisation, les particules peuvent être obtenues par auto-assemblage des protéines ou peptides. Dans de tels modes de réalisation, les particules ne comprennent pas de support, elles consistent uniquement en des protéines ou peptides. L'auto-assemblage peut être réalisé par différent moyen, tel que par coacervation ou par agrégation, par exemple par précipitation, concentration, ajouts de métaux ou de sels, variation de température.

Les particules de la présente invention peuvent avoir un diamètre moyen allant de 0.05 µm à 6 µm, ou de 1 à 5 µm ou de 2 à 4 µm tel que mesuré par diffusion dynamique de la lumière (DLS). De manière avantageuse, le diamètre moyen des particules de la présente invention peut être compris entre 0,02 µm et 0,05 µm, par exemple compris entre 0,025 et 0.045, ou par exemple égal à 0,035 µm.

Lorsque que les particules comprennent ou consistent en des protéines recombinantes dont la séquence inclut au moins un polypeptide choisi parmi :
- le polypeptide de la séquence ID n°1 ;
- le polypeptide ayant la séquence de la position 25 à 184 de la séquence ID n°1 ; ou
- un polypeptide présentant au moins 70% d'identité sur toute sa longueur avec le polypeptide de la séquence ID n°1 ou avec le polypeptide ayant la séquence de la position 25 à 184 de la séquence ID n°1 ;
et lorsqu'elles présentent un diamètre moyen supérieur à 2 µm tel que mesuré par DLS, elles sont capables d'induire l'adhésion, l'activation et l'agrégation des plaquettes. Cet effet est également trouvé pour des molécules dont le diamètre moyen est inférieur à 2µm, par exemple compris entre 0,02 µm et 1 µm, entre 0,05 µm et 0,5 µm, ou encore entre 0,1 µm et 0,3µm.

### Véhicule ou excipient pharmaceutiquement acceptable

Des véhicules ou des excipients pharmaceutiquement acceptables selon l'invention, c'est à dire des véhicules ou des excipients dont l'administration à un individu ne s'accompagne pas d'effets délétères significatifs, sont bien connus de l'homme du métier.

De manière non limitative, des exemples d'excipients ou véhicules pharmaceutiquement acceptables incluent les solvants, agents d'écoulement, agents de mise en suspension, agents de solubilisation, stabilisants, conservateurs, tampons, antioxydants et agents chélatants.

Les préparations injectables selon la présente invention peuvent être préparées selon des méthodes bien connues de l'homme du métier.

Les préparations injectables selon la présente invention sont capables de contrôler les saignements éventuellement par activation des plaquettes et/ou de se substituer à des plaquettes dans le cas des syndromes hémorragiques.

La présente description divulgue des méthodes de traitement des hémorragies comprenant l'administration par injection dans la circulation sanguine d'un individu d'une quantité efficace d'une préparation telle que définie ci-dessus.

Ainsi, il est clair que dans le contexte de la présente invention, une « injection » est synonyme d'une administration systémique, ou encore dite administration générale, du produit de l'invention à un patient.

La présente description divulgue, enfin, l'utilisation d'une préparation telle que définie ci-dessus pour la préparation d'un médicament pour traiter les hémorragies.

### EXEMPLES

### Production d'un collagène et auto-assemblage en particules (méthode de détermination de la taille des particules par DLS)

Une pré-culture des cellules CHO-S (Invitrogen) de 3 semaines est réalisée avant la transfection. Les cellules sont entretenues dans un milieu dédié aux cellules CHO (Power-CHO, EXCEL 302, proCHO4, proCHO5, ...) complémenté avec 4 mM de L-glutamine (Lonza) et 1X de proHT (Lonza) en shakeflask 125 mL et en conditions agitées (80 rpm) dans un incubateur à 37°C avec 5% de CO₂. Deux jours avant la transfection, les cellules sont ensemencées à 5.10⁵ cellules viables/mL par un changement complet du milieu utilisé et cultivées dans 12,5 mL de milieu dédié aux cellules CHO complémenté en shakeflask 125 mL.
Le jour de la transfection, 5.10⁶ cellules viables sont culotées par centrifugation (5 min à 1000 g), puis reprises dans 5 mL de milieu RPMI (Lonza) complémenté avec 4 mM de L-Glutamine (Lonza) et 1X de ProHT (Lonza). Quatre mL de suspension sont alors répartis dans 4 shakeflasks 25 mL (1 mL par flask) contenant 9 mL de milieu RPMI complémenté (1.10⁶ cellules viables par shakeflask). Les cellules CHO-S sont alors transfectées avec le vecteur contenant la séquence codante pour le polypeptide de SEQ n°1 décrit précédemment. Un contrôle positif de transfection est réalisé en transfectant les cellules avec le vecteur pMAX-GFP et 2 contrôles négatifs de la transfection sont réalisés soit en transfectant les cellules avec un vecteur ne possédant pas la cassette de résistance à la généticine soit en ne leur faisant subir aucun traitement. La transfection est réalisée à l'aide de l'agent de transfection Fecturine (PolyPlus Transfection) ou tout autre agent de transfection adapté et selon le protocole commercial du produit optimisé. Pour la Fecturine, les conditions de transfection retenues sont 6 µg d'ADN pour 12 µL de Fecturine (ratio ADN/Agent de transfection = ½) pour 10⁶ cellules viables. L'homme de métier saura définir l'agent de transfection le plus adapté pour une transfection dite transitoire ou pour une transfection dite stable. Qu'il s'agisse d'un mode de transfection dit transitoire ou stable, les cellules sont incubées en présence des complexes de transfection en conditions statiques à 37°C et 5% CO₂. A 4h post-transfection, les cellules sont resuspendues dans du milieu dédié aux cellules CHO complémenté et repassées en conditions agitées. A 24h post-transfection, une quantification de l'efficacité de transfection est réalisée sur un aliquot des témoins positif et négatifs de transfection par cytométrie en flux.
Pour la production du polypeptide de SEQ n°1 en mode dit transitoire, un premier prélèvement de surnageant est réalisé à J+3 (J0 correspondant au jour de la transfection) puis la culture est stoppée à J+5. Le surnageant de culture contenant le polypeptide de SEQ n°1 sécrété par les cellules est récupéré après centrifugation à 3000 g pendant 10 minutes, permettant ainsi l'élimination des cellules et débris cellulaires, puis congelé à -20°C.
Pour la production du polypeptide de SEQ n°1 en mode dit stable, un comptage cellulaire est réalisé à 48 heures post-transfection. La totalité des cellules est ensuite centrifugée à 1000 g pendant 5 min puis ensemencée à 3.10⁵ cellules viables/mL dans du milieu dédié aux cellules CHO complémenté + généticine (G418 Merck) à 700 µg/mL (pression de sélection). Les cellules sont ensuite entretenues trois fois par semaine par ensemencement de la totalité des cellules dans 12,5 mL final de milieu complet + G418 à 700 µg/mL en ShakeFlasks 125 mL. On observe une diminution de la concentration cellulaire et/ou de la viabilité cellulaire dans la première semaine de culture sous pression de sélection. La viabilité cellulaire augmente de nouveau après 2-3 semaines sous pression de sélection uniquement pour les cellules transfectées avec le vecteur contenant la séquence codante pour le polypeptide de SEQ n°1 ou le vecteur vide. La viabilité des cellules non transfectées continue de descendre jusqu'à devenir nulle. Lorsque la culture atteint plus de 95 % de viabilité, une cryo-conservation à 5.10⁶ cellules viables/ampoule (au nombre de 10 ampoules) est réalisée. Les cellules sont congelées dans du milieu dédié aux cellules CHO complémenté + 10% de DMSO.
Pour réaliser une production du polypeptide de SEQ n°1, les cellules modifiées génétiquement pour produire le polypeptide de SEQ n°1 sont décongelées et entretenues dans un milieu adapté. Les cellules sont placées dans 12,5 mL de milieu final et placées dans un shakeflask 125 mL dans un agitateur LabTherm® Kühner agité à 80 rpm, régulé à 5% de CO₂ et avec une humidité comprise entre 40 et 80 %. Les cellules sont entretenues à 3.10⁵ cellules viables/mL pendant une à deux semaines. Ensuite, les cellules sont amplifiées afin d'avoir la quantité nécessaire pour réaliser une production. L'amplification consiste à entretenir les cellules dans des volumes plus élevés à chaque entretien pour garder toutes les cellules à une concentration viable.
Lorsque la quantité de cellules nécessaire à la production est obtenue, la production peut être lancée. Les cellules sont ensemencées à 3.10⁵ cellules viables/mL. La production peut être réalisée dans différents équipements : shakeflask placés dans un agitateur LabTherm® Kühner, Cultibag RM 20/50® (Sartorius), CellReady® (Merck Millipore), BioBundle® (Applikon) et d'autres équipements équivalents, de même échelle ou d'échelle supérieure. La culture des cellules est réalisée pendant 5 jours ou plus, au maximum 10 jours, selon les conditions de réalisation de la culture. Chaque jour, les paramètres de la production sont suivis ainsi que la concentration cellulaire et la viabilité cellulaire. Au cours de la production, des composants tels que des acides aminés, des vitamines, du glucose ou tout autre élément présentant un intérêt pour la production ou pour les cellules peuvent être ajoutés. Dans ce cas, il s'agit d'une culture en mode « fed - batch » ou « semi continu » qui permet de cultiver les cellules pendant au maximum 21 jours. Si aucun composé n'est ajouté pendant la culture, on parle de culture en mode « batch » ou « discontinu ».
La purification du polypeptide de SEQ n°1 peut être réalisée par exemple via une étiquette de purification comme le tag-6(His) présent dans sa séquence. Ainsi, les cellules et débris cellulaires présents dans le milieu contenant le polypeptide de SEQ n°1 sont éliminés par centrifugation ou filtration en profondeur (POD Millistak+® Merck Millipore ou tout un autre type de support équivalent) ou filtration tangentielle sur une membrane présentant un seuil de coupure de 0,2 µm. Le surnageant ainsi obtenu est purifié par chromatographie d'affinité sur une colonne chélatée avec un métal tel que le nickel, le cobalt, le zinc ou le cuivre. Afin de favoriser l'accroche du polypeptide de SEQ n°1, un tampon contenant de 0 à 50 mM imidazole, de 0 à 500 mM NaCl, de 5 à 20 mM (Na₂HPO₄, 2H₂O) et de 5 à 20 mM (NaH₂PO₄, H₂O) est ajouté au surnageant et ajusté à un pH entre 7 et 8. L'élution du polypeptide selon l'invention est soit réalisée par gradient en utilisant un mélange de deux tampons (tampon 1: 500 mM imidazole, 500 mM NaCl, 10 mM (Na₂HPO₄, 2H₂O) et 10 mM (NaH₂PO₄, H₂O); tampon 2 : 20 mM imidazole, 500 mM NaCl, 10 mM (Na₂HPO₄, 2H₂O) et 10 mM (NaH₂PO₄, H₂O)), soit de façon isocratique, avec un tampon contenant de 50 à 500 mM imidazole, de 0 à 500 mM NaCl, de 5 à 10 mM (Na₂HPO₄, 2H₂O) et de 5 à 10 mM (NaH₂PO₄, H₂O). Eventuellement, afin d'améliorer la pureté du polypeptide de SEQ n°1, d'autres étapes de purification peuvent être ajoutées. Il peut s'agir de filtrations, de chromatographie d'échange d'ions, de chromatographie d'affinité, de chromatographie d'interaction hydrophobe, d'exclusion stérique ou de tout autre type de chromatographie. Les fractions d'élution d'intérêt sont mises à migrer dans un gel d'électrophorèse en conditions natives et colorées au bleu de Coomassie, avant d'être regroupées selon leur profil et dialysées dans de l'eau ou un tampon phosphate ou tout autre tampon adapté à la conservation du polypeptide selon l'invention. La concentration du polypeptide est déterminée par le kit Sircol® (TebuBio) selon les instructions du fabricant ou par le test au Bradford ou tout autre test adapté à la quantification du polypeptide de SEQ n°1.
Le polypeptide de SEQ n°1 est concentré plusieurs fois (50 fois) par rapport à son volume initial par ultrafiltration, permettant d'atteindre une concentration protéique suffisante pour induire l'auto-assemblage du polypeptide de SEQ n°1 et la formation de particules. L'extrait protéique contenant le polypeptide de SEQ n°1 est ainsi concentré sur des colonnes d'ultrafiltration ayant un cut-off de 10 000 Da (Vivaspin 4 ; Sartorius) par des centrifugations répétées réalisées à 4°C et à 4 000 rpm. Une fois la solution concentrée, une mesure de la taille des particules ainsi formées est réalisée par la technique de diffusion dynamique de la lumière (DLS) à l'aide d'un appareil de la série Zétasizer nano de chez Malvern. La mesure en DLS est une technique d'analyse spectroscopique non destructive permettant d'accéder à la taille de particules en suspension dans un liquide particulièrement adapté pour la mesure de l'auto-assemblage ou de l'agrégation de protéines comme le polypeptide de SEQ n°1.

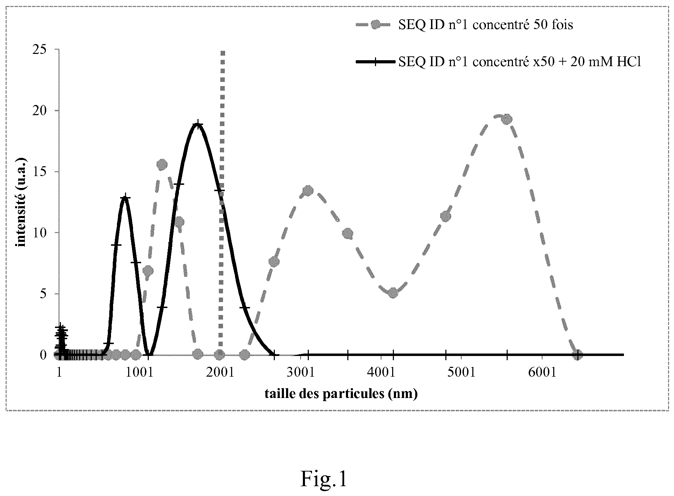

La figure 1 présente un résultat caractéristique de mesure de la taille des particules obtenu par DLS après concentration d'un facteur 50 d'un extrait protéique du polypeptide de SEQ n°1. Une série de pics correspondant à différentes tailles de particules présentes dans la solution est observée. Ainsi, trois pics sont majoritaires. Ils correspondent à des tailles de particules de 1,25 ; 3 et 5,5 µm. Afin de relier l'activité biologique du polypeptide de SEQ n°1 à la taille des particules ainsi générées, 20 mM finaux d'acide chlorhydrique sont ajoutés à la solution. On observe alors la disparition des trois pics préalablement mesurés et l'apparition de 2 pics à environ 500 nm et 1.8 µm. Visuellement, on observe un « éclaircissement » de la fraction qui passe d'un aspect granuleux à un aspect translucide.

### Mesure de l'activité de liaison au facteur Willebrand en fonction de la taille des particules

La préparation à base du polypeptide de SEQ n°1 sous forme de particules possède la capacité à lier le facteur Willebrand. La mesure de l'activité de liaison du facteur Willebrand au polypeptide de SEQ n°1 par la technique dite ELISA est réalisée dans des plaques 96 puits (Nunc Maxisorp). Pour cela, un volume de 100 µL d'une solution contenant le polypeptide à 2 µg/mL dans du tampon phosphate ou tout autre tampon adapté est ajouté dans chaque puits et incubé 18-20h à 22°C. Après trois lavages avec 200 µL de PBS-0,05% Tween, 200 µL d'une solution de BSA 1% dans du tampon phosphate (Euromedex, filtrée sur 0,45 µm) sont ajoutés dans chaque puits et incubés 2h à température ambiante (22 °C). Après trois lavages avec 200 µL de PBS-0,05% Tween, 100 µL de différentes concentrations (exprimées en UI/dL) de facteur Willebrand purifié (Wilfactin 100 UI/mL, LFB) et/ou de plasma de patient dilué dans du tampon phosphate ou tout autre tampon adapté sont incubés 1h30 à température ambiante (22°C). Après trois nouveaux lavages, 100 µL d'une solution contenant un anticorps primaire anti-vWF couplé à la peroxydase de raifort (Rabbit anti-human VWF/HRP, DAKO) dilué au 1/8 000^{e} dans du tampon phosphate ou tout autre tampon adapté sont incubés 1h à 22°C puis 4 lavages sont réalisés comme décrit ci-dessus. 125 µL de solution de tétraméthylbenzidine sont ensuite ajoutés comme substrat pour la peroxydase (TMB, Sigma) puis on incube 10 à 45 minutes maximum à l'obscurité. La réaction est interrompue par l'ajout de 125 µL d'HCl 2N (Sigma). L'absorbance est rapidement mesurée à 450 nm dans un spectrophomètre (Wallacvictor 3). Un blanc est réalisé en remplaçant le vWF purifié ou le plasma par du tampon phosphate ou tout autre tampon adapté.

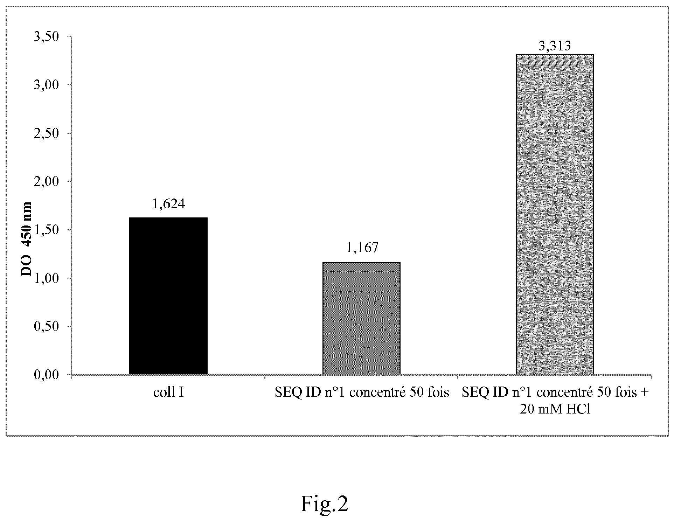

La figure 2 présente un résultat obtenu par ELISA de la liaison du facteur Willebrand purifié (Willfactin, LFB) utilisé à 2 UI/dL final aux polypeptides de SEQ n°1 sous forme de particules de différentes tailles, utilisés à 2 µg/mL final et au collagène de type I (Collagène SIRCOL, contrôle positif) utilisé à 10 µg/mL.

Les résultats montrent que le polypeptide de SEQ n°1, formé majoritairement de particules de taille supérieure à 2 µm, est capable de lier le facteur Willebrand de façon équivalente au collagène de type I. L'activité de liaison au facteur Willebrand est fortement augmentée lorsque le polypeptide de SEQ n°1 est sous forme de particules de taille inférieure à 2 µm. Ce dernier résultat suggère que l'auto-assemblage du polypeptide de SEQ n°1 en particules de plus haute taille diminue le nombre de sites de reconnaissance au facteur Willebrand, ces derniers étant probablement « masqués » à l'intérieur des particules de haute taille. Ce résultat montre qu'un polypeptide de SEQ n°1 structuré en particules de taille comprise entre 1 et 5 µm est bien capable de lier le facteur Willebrand.

### Mesure de l'expression de surface de la P-sélectine induite par du collagène en fonction de la taille des particules

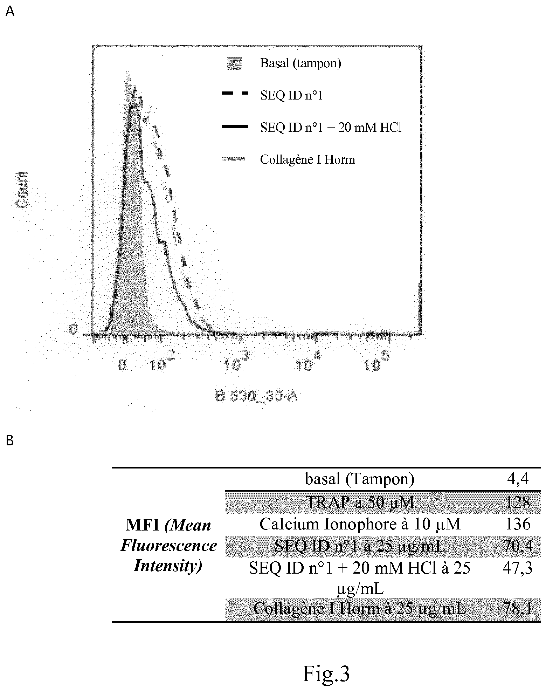

La préparation à base du polypeptide de SEQ n°1 sous forme de particules possède la capacité d'induire l'activation des plaquettes. Cette activation peut être déterminée en sang total en mesurant par cytométrie en flux l'expression en surface des plaquettes de la P-sélectine. Dans des plaquettes au repos, cette dernière est stockée dans des granules, dits grains alpha. Lors de l'activation des plaquettes par différents agonistes, la P-sélectine est déchargée de ces granules et elle est détectable en surface des plaquettes.
Un prélèvement de sang est réalisé sur tube citraté (BD Vacutainer, BD Biosciences) puis stabilisé 1h avant d'être utilisé. Dans des tubes en verre, un volume de 50 µL de sang est ajouté à un volume de 50 µL d'une solution de tampon phosphate contenant le polypeptide de SEQ n°1 à 50 µg/mL (soit 25 µg/mL en final) puis le tout est incubé 10 minutes à température ambiante.
La réaction est ensuite stabilisée par ajout de 500 µL de tampon phosphate dans chaque tube. Dans des tubes de cytométrie, un volume de 20 µL du mix réactionnel précédemment décrit est ensuite ajouté à 20 µL d'une solution contenant soit un anticorps primaire anti-CD62P couplé à la FITC (FITC Mouse Anti-Human CD62P, *BD Biosciences)* soit un anticorps isotype contrôle couplé à la FITC (FITC Mouse IgG1 κ isotype control, *BD Biosciences;)* et est incubé 10 minutes à température ambiante dans l'obscurité. Un volume de 2 mL de tampon phosphate est ensuite ajouté dans chaque tube. La moyenne de fluorescence (MFI ; Mean Fluorescence Intensity) est rapidement mesurée à 520 nm à l'aide d'un cytomètre en flux (LSRII, BD Biosciences). Un contrôle négatif est réalisé en remplaçant le polypeptide de SEQ n°1 par du tampon phosphate. Deux contrôles positifs sont réalisés en remplaçant le polypeptide de SEQ n°1 par l'activateur plaquettaire dérivé de la thrombine TRAP (kit de diagnostique clinique humain: PLT Gp/Receptors Kit *(Biocytex)* ou l'activateur ionophore A23187 *(Calbiochem (Merck); Streptomyces chartreusensis).* Un référent est réalisé en remplaçant le polypeptide par du collagène de type I utilisé à 50 µg/mL (Horm). La figure 3A correspond à une représentation d'un profil type de l'expression de la P-sélectine en surface de plaquettes obtenu en cytométrie en flux pour le polypeptide de SEQ n°1 et du collagène de type I (Horm), collagène de référence. Le tableau de la figure 3B regroupe les intensités moyennes de fluorescence obtenues pour différents agonistes. Le TRAP et le calcium ionophore sont utilisés comme contrôles positifs d'activation.
Le polypeptide de SEQ n°1 présentant des tailles de particules supérieures à 2 µm est capable d'induire l'activation des plaquettes caractérisée par l'expression en surface de la P-sélectine. Cette activation est équivalente à celle induite par le collagène de type I fibrillaire (fibres supérieures à 1 µm), utilisé à la même concentration. Le polypeptide de SEQ n°1 présentant des tailles de particules inférieures à 2 µm (SEQ N°1 + 20 mM HCl) est également capable d'induire l'expression de la P-sélectine et donc l'activation des plaquettes mais de façon moins importante (47.3 vs. 70.4 MFI).

### Mesure de l'agrégation plaquettaire induite par du collagène en fonction de la taille des particules

La préparation à base du polypeptide de SEQ n°1 sous forme de particules possède une activité pro-agrégante sur les plaquettes sanguines humaines détectée à l'aide d'un thrombo-agrégomètre (société Soderel, Florange, France), selon la technique de référence (Born 1962). Le plasma riche en plaquettes (PRP) est mis en contact avec un agoniste (10 µL dans 290 µL de PRP) et l'éclaircissement du milieu (lié à la formation d'agrégats) est suivi en temps réel (courbe d'agrégation). En l'absence d'agrégation plaquettaire, le signal reste plat (milieu trouble persistant). Il est possible de vérifier l'absence ou la présence d'agrégats à l'examen du tube à la fin de la mesure. L'évaluation de la réponse en test d'agrégation plaquettaire est effectuée à 37°C, en agitation continue (1000 rpm). L'appareil est étalonné comme suit : 0% d'agrégation avec le plasma riche en plaquettes (préparation obtenue par centrifugation lente et concentration ajustée à 300 x 10⁹ / L) et 100% d'agrégation avec le plasma pauvre en plaquettes (préparation obtenue par centrifugation rapide). La qualité des préparations plaquettaires est validée en vérifiant la réponse à des agonistes de référence (ADP 5 µM et collagène 1 µg/mL), utilisés pour la mise au point des thérapeutiques antiplaquettaires. Une protéine est considérée pro-agrégante quand elle capable d'induire une agrégation supérieure à 40% et ce, de façon irréversible.

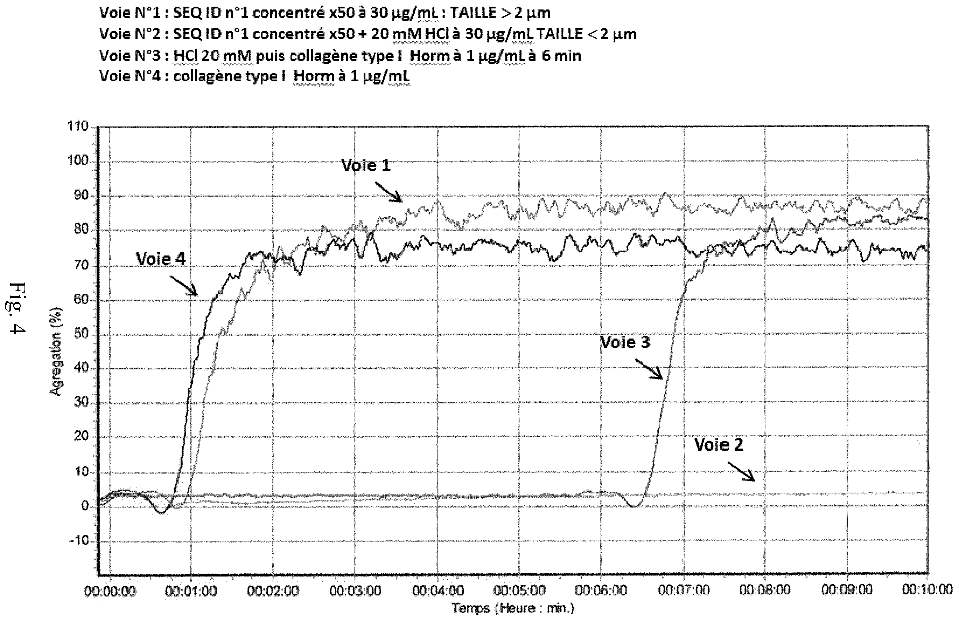

La figure 4 présente un résultat d'agrégation obtenu avec le polypeptide de SEQ n°1 sous forme de particules. La voie 1 montre que la préparation du polypeptide de SEQ n°1 présentant des tailles de particules supérieures à 2 µm est capable d'induire l'agrégation des plaquettes en moins de 1 minute et de façon équivalente au collagène de type I Horm (voie 4), collagène de référence de forme fibrillaire. A l'inverse, le polypeptide de SEQ n°1 présentant des tailles de particules inférieures à 2 µm n'est pas capable d'induire l'agrégation des plaquettes (voie 2) même après 10 minutes. Cette absence d'activité n'est pas lié à la présence dans la solution de 20 mM de HCl car la voie 3 montre une agrégation normale des plaquettes lorsqu'elles sont stimulées par du collagène de type I, ajouté après 6 minutes, en présence de 20 mM de HCl.
Ce résultat montre que pour obtenir une activité pro-agrégante le polypeptide de SEQ n°1 doit être structuré sous forme de particules de tailles supérieures à 2 µm.
En conclusion, ces différents exemples ont montré que le polypeptide de SEQ n°1 est capable de se structurer sous forme de particules de taille comprise entre 1 et 6 µm, que ces particules possèdent la capacité de se lier au facteur Willebrand, d'induire l'activation des plaquettes (expression de surface de la P-sélectine) et, uniquement lorsque la taille des particules est supérieure à 2 µm, de provoquer l'agrégation des plaquettes..

### Observation par différentes techniques de microscopie de la structuration du polypeptide de la SEQ n°1 sous forme de particules en comparaison du collagène de type I (Horm) fibrillaire.

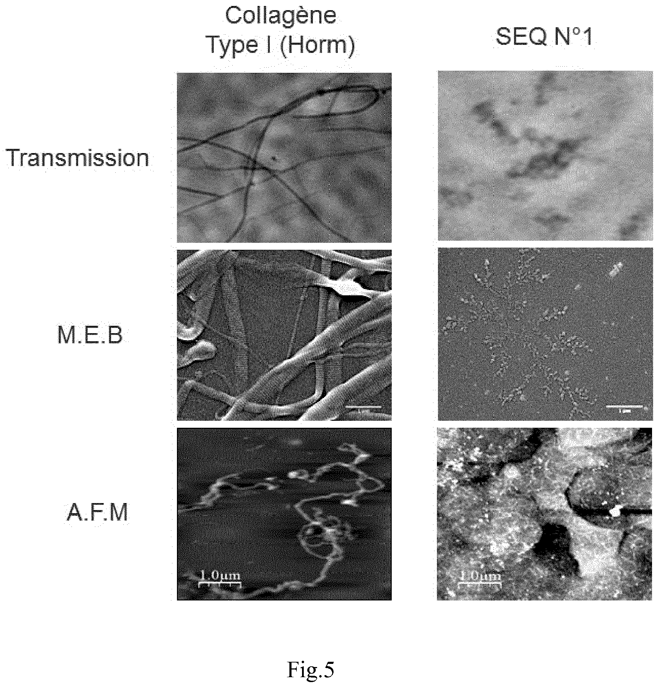

Il existe différentes techniques de microscopie qui permettent de bien visualiser la structure macromoléculaire d'une molécule, et ce, à différentes résolutions et échelles. La figure 5 présente des images obtenues avec trois techniques de microscopie (microscopie à transmission, à balayage et à force atomique) permettant l'observation de la structuration de protéines de type collagène.

Pour l'observation par microscopie à transmission, le collagène de type I ou le polypeptide de SEQ n°1 est déposé sur une lame de verre pendant 2 heures à 37°C avant d'être observé à l'aide d'un microscope inversé à contraste de phase de type ZEISS à l'objectif X60. Les images sont obtenues à l'aide d'une caméra ZEISS AxioCam ICc 1.

Pour l'observation par microscopie à balayage (MEB), la surface choisie pour le dépôt est le silicium monocristallin (100). Étant à la fois semi-conducteur et atomiquement plan, c'est un choix évident pour l'observation des collagènes par cette modalité. La préparation de l'échantillon a été réalisée en déposant pendant 6 heures sur la surface de silicium une goutte de 10 µL de collagène de type I fibrillaire ou du polypeptide de SEQ n°1 concentré à 200 µg.mL⁻¹. La surface est ensuite rincée à l'eau distillée puis séchée à l'azote. La surface séchée est métallisée par sputtering d'or sur un pulvérisateur Edwards S150 (intensité de 25 mA en atmosphère Argon à 0,3 mPa pendant 2 minutes). L'observation a lieu avec un microscope électronique à balayage JEOL 6500F, avec une tension d'émission de 20 keV et un courant d'émission de 60 µA.

Pour l'observation par microscopie à force atomique (AFM), la surface sur laquelle le collagène de type I et le polypeptide de SEQ n°1 sont observés est une surface d'or polycristallin (terrasses d'Au(111) obtenue par épitaxie sur mica, PHASIS, réf. 20020022). Le dépôt a eu lieu ex situ à une concentration de 25 µg.mL⁻¹ sous flux à un taux de cisaillement de 300 s⁻¹ pendant 30 minutes. Pour permettre cette fonctionnalisation en flux, une cellule microfluidique en PDMS est pressée sur la surface d'or et la solution de protéines est amenée jusqu'à cette surface via une pompe péristaltique ISMATEC IPC-4. Les échantillons ont été observés avec un microscope AFM Nanoscope IV Multimode (Digital Instrument, Veeco Inc., Santa Barbara, CA), équipé de pointes FESPA Silicium (Bruker) avec une constante de raideur k d'un N.m⁻¹, une fréquence de résonance f = 50 - 100 kHz et une longueur de levier L = 200 - 250 µm. L'acquisition est faite avec le mode PeakForce QNM de Bruker, un mode tapping dynamique permettant une acquisition peu bruitée sur ces échantillons biologiques. La fréquence de balayage est fixée à 1 Hz. Les images obtenues ont été traitées numériquement afin d'obtenir une meilleure résolution via le logiciel WSxM, en appliquant les fonctions d'aplatissement (flatten) et d'égalisation (equalize).

Les observations présentées dans la figure 5 confirment les résultats obtenus en DLS indiquant une structuration sous forme de particules pour le polypeptide de SEQ n°1, en comparaison du collagène de type I de structure fibrillaire. La taille des particules observées est de l'ordre de quelques dizaines de nanomètres dans les conditions expérimentales utilisées pour leur observation.

### Exemple comparatif de mesure de l'expression de surface de la P-sélectine plaquettaire après injection chez la souris du polypeptide de SEQ n°1 sous forme de particules et du collagène de type I (Horm) sous forme fibrillaire.

La préparation à base du polypeptide de SEQ n°1 sous forme de particules ou de collagène de type I fibrillaire possède ex vivo, sur sang total, la capacité d'induire l'activation des plaquettes, révélée par l'expression en surface des plaquettes de la P-sélectine mesurée par cytométrie en flux. Afin de valider que ces préparations sont bien capables d'induire cette même activation une fois injectées dans la circulation sanguine, un prélèvement de sang est réalisé sur tube citraté (BD Vacutainer, BD Biosciences) 15 minutes après administration chez la souris de 400 µg/kg de collagène de type I et 2 mg/kg du polypeptide de SEQ n°1, ou au moment des premières manifestations d'une embolie (suffocation). Un volume de 50 µL de sang est ajouté dans des tubes en verre puis stabilisé avec 250 µL de tampon phosphate. Dans des tubes de cytométrie, un volume de 20 µL du mix réactionnel précédemment décrit est ensuite ajouté à 20 µL d'une solution contenant soit un anticorps primaire anti-CD62P couplé à la FITC (FITC Mouse Anti-Human CD62P, *BD Biosciences*) soit un anticorps isotype contrôle couplé à la FITC (FITC Mouse IgG1 κ isotype control, *BD Biosciences;)* et est incubé 10 minutes à température ambiante dans l'obscurité. Un volume de 2 mL de tampon phosphate est ensuite ajouté dans chaque tube. La moyenne de fluorescence (MFI ; Mean Fluorescence Intensity) est rapidement mesurée à 520 nm à l'aide d'un cytomètre en flux (LSRII, BD Biosciences).

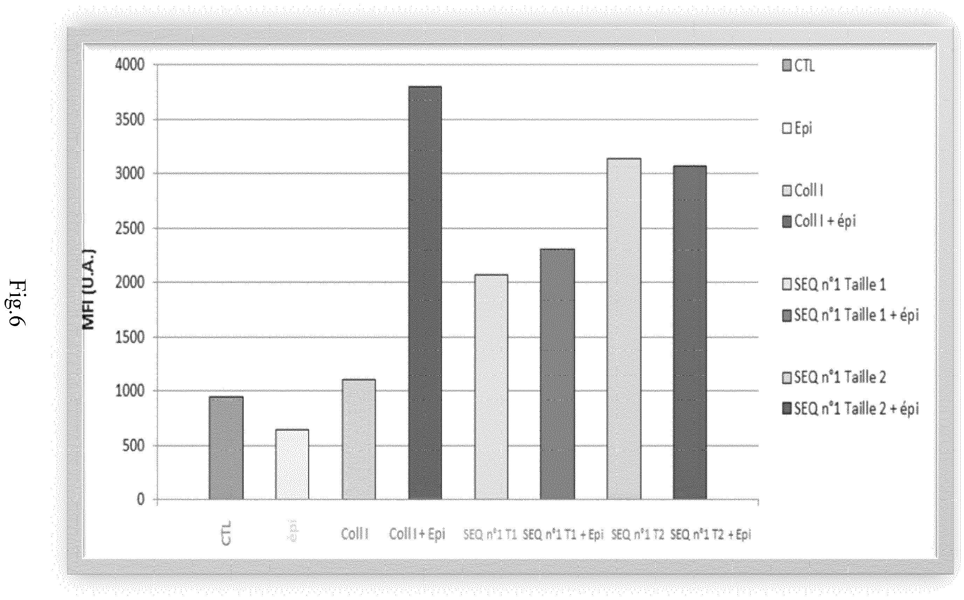

La figure 6 présente l'expression plaquettaire de la P-sélectine mesurée à partir de sang prélevé chez des souris après injection de collagène de type I fibrillaire et du polypeptide de SEQ n°1, pour deux tailles de particules, en présence ou non d'épinéphrine. On constate que le polypeptide de SEQ n°1 induit bien l'activation des plaquettes 15 minutes après son administration chez la souris ou au moment des premières manifestations d'une embolie (suffocation), quelle que soit la taille des particules. Cette activation n'est pas modulée par l'administration concomitante d'épinéphrine, contrairement au collagène de type I fibrillaire dont l'activation est fortement augmentée par cette dernière. Ce résultat confirme bien que le polypeptide de SEQ n°1 est bien capable d'induire l'activation des plaquettes une fois administré dans la circulation sanguine comme démontré *ex vivo.*

### Mesure du risque thrombotique associé à l'injection dans la circulation sanguine du polypeptide de SEQ n°1 dans un modèle d'embolie pulmonaire induite chez la souris

La présente description divulgue l'utilisation de protéines ou peptides dérivés du collagène pour le traitement des hémorragies par un mécanisme dépendant de l'activation des plaquettes. Afin de déterminer si l'activation *in vivo* des plaquettes induites par l'injection de ces protéines ou peptides dérivés du collagène sous forme de particules est associée, indépendamment d'un contexte hémorragique, à un risque de thrombose, un modèle d'embolie pulmonaire induite chez la souris par administration de collagène et d'épinéphrine a été utilisé. L'objectif est de démontrer que l'activation des plaquettes induite par les protéines ou peptides dérivés du collagène de la présente invention ne sont pas par eux-mêmes délétères en induisant la formation de thrombus une fois injectés. Afin de bien caractériser l'effet potentiellement thrombotique de cette administration, un traceur radioactif visualisable par imagerie isotopique (SPECT) a été utilisé comme critère observationnel, en plus de celui classiquement utilisé dans ce modèle et consistant à mesurer le temps au bout duquel l'animal décède d'embolie pulmonaire. Après injection intraveineuse, les macroagrégats d'albumine humaine marqués au technétium-99m circulent dans le sang et permettent de réaliser des scintigraphies des poumons ou de certaines veines. Si des foyers thrombotiques existent, la distribution pulmonaire normale de ce traceur sera perturbée et l'imagerie isotopique révèlera la diminution ou l'absence du traceur au niveau pulmonaire.

Des souris SWISS mâles de 25 à 30 g sont utilisées pour réaliser le modèle d'embolie pulmonaire. Les souris sont maintenues sous anesthésie à l'isoflurane pendant toute la durée de l'expérimentation. Dans un premier temps, les deux jugulaires sont mises à nu avant d'injecter, dans l'une d'elles, le mélange polypeptide de SEQ n°1 (1 mg/kg) / épinéphrine (60 µg/kg) ou collagène de type I fibrillaire (400 µg/kg) / épinéphrine (60 µg/kg) dans un volume final de 150 µL. Si une embolie pulmonaire se produit, les souris se mettent à suffoquer et meurent dans les 10 minutes suivant l'injection. On considère que les souris survivant au-delà de 15 minutes ne font pas d'embolie pulmonaire. Pour réaliser une acquisition d'image en SPECT, les macroagrégats d'albumine humaine marqués au technétium-99m (30 µCi par souris dans 100 µL finaux) sont injectés dans la deuxième jugulaire juste avant l'arrêt cardiaque pour les souris réalisant une embolie pulmonaire ou au bout de 15 minutes pour les souris qui survivent. Un scanner puis une acquisition de 15 minutes en imagerie isotopique sont réalisés.

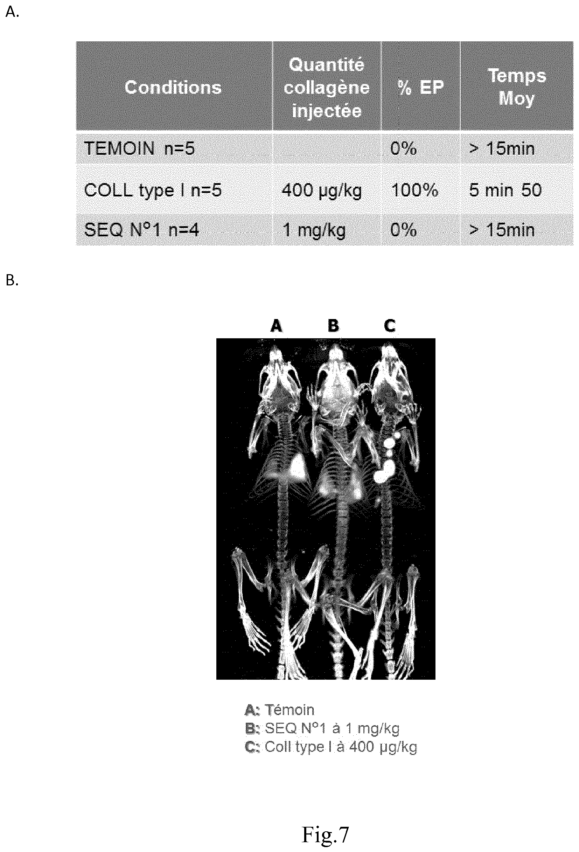

Le tableau de la figure 7A présente les résultats obtenus, après administration chez la souris de collagène de type I fibrillaire et du polypeptide de SEQ n°1 sous forme de particules en présence d'épinéphrine, du % d'animaux chez lesquels une embolie pulmonaire a été observée et le temps moyen pour l'installation de cette embolie. Les résultats montrent que 100% des animaux sont morts au bout de 5 min 50 en moyenne après injection du collagène de type I fibrillaire. A l'inverse, aucun animal n'a développé d'embolie pulmonaire après injection du polypeptide de SEQ n°1 particulaire, pourtant utilisé 2,5 fois plus concentré (1 mg/kg vs. 400 µg/kg). La figure 7B présente les images obtenues après injection de macroagrégats d'albumine radiomarqués qui confirment la présence d'une embolie pulmonaire massive chez les souris injectées avec du collagène de type I fibrillaire et la distribution pulmonaire normale du traceur chez les souris injectées avec le polypeptide de SEQ n°1, signe de l'absence de thrombose induite.

Ce résultat confirme que pour deux protéines de type collagène possédant la même activité activatrice plaquettaire une fois injectées dans la circulation sanguine, seule celle sous la forme de particules peut être utilisée sans risque d'induire une thrombose.

### Mesure de l'effet de l'injection du polypeptide de SEQ n°1 dans un modèle de saignement induit à la queue chez la souris.

Les résultats présentés précédemment confirment que le polypeptide de SEQ n°1 sous forme de particules est bien capable d'induire l'activation des plaquettes une fois administré dans la circulation sanguine de souris et que cette activation n'a pas d'effet thrombogène, contrairement à du collagène de type I fibrillaire. Afin de valider que l'injection dans la circulation sanguine d'une protéine ou d'un peptide dérivé du collagène de la présente invention permet bien d'induire l'arrêt de saignements par un mécanisme dépendant de l'activation des plaquettes, l'effet sur la perte sanguine de l'administration du polypeptide de SEQ n°1 a été évalué dans un modèle de saignement induit à la queue chez la souris.

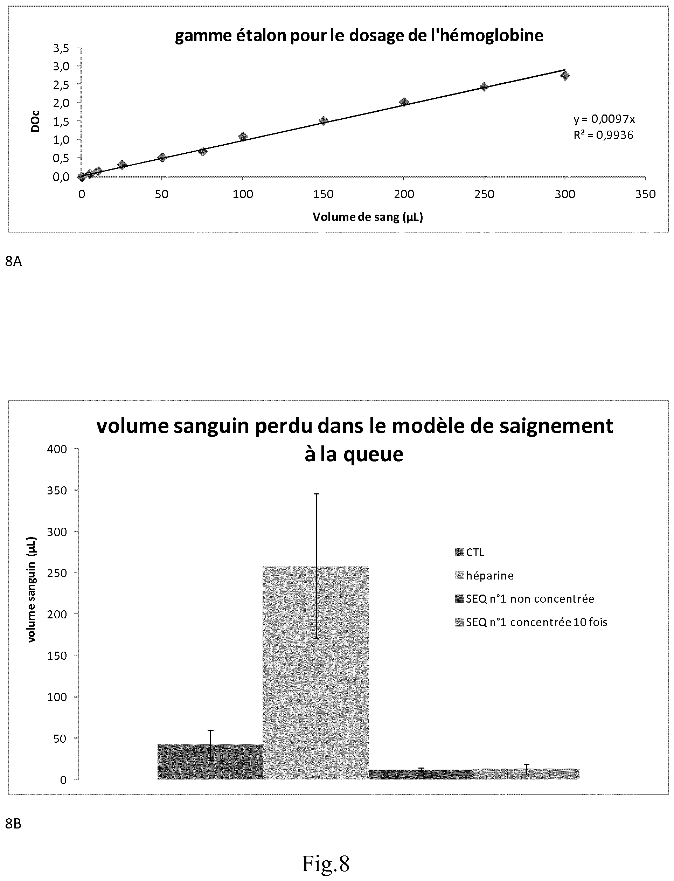

Des souris SWISS mâles de 25 à 30 g sont utilisées pour réaliser le modèle de saignement à la queue. Les souris sont maintenues sous anesthésie à l'isoflurane pendant toute la durée de l'expérimentation. Dans un premier temps, une des deux jugulaires est mise à nu avant d'injecter les différentes molécules diluées dans un tampon phosphate. Une section de la queue est réalisée à 1.5 cm du bout de la queue afin de sectionner les 3 artères et la veine. La section est réalisée 10 minutes après injection du tampon phosphate (contrôle véhicule) ou du polypeptide de SEQ n°1 (1.6 mg/kg) et 20 minutes après injection d'héparine (anti coagulant - contrôle positif de saignement - 60 U/kg). Après coupure, la queue est plongée dans 50 mL d'eau physiologique à 37°C afin de récolter le sang pendant 16 minutes sans abimer les globules rouges. Les tubes sont laissés à décanter pendant 1 heure à température ambiante avant d'être centrifugés 15 minutes à 250g. Le surnageant est éliminé et le culot de globule rouge est repris dans 3 mL de tampon de lyse spécifique (8.3 g/L NH4Cl, 1 g/L KHCO3 et 37 mg/L EDTA). La densité optique de 200 µL de lysat est lue à 550 nm sur un lecteur de plaque.
Une courbe étalon a été réalisée afin de déterminer le volume de sang perdu. Pour cela, des souris SWISS mâles de 25 à 30 g ont été exanguinées au niveau de l'aorte abdominale. Le sang a été récolté dans des tubes citratés afin d'éviter que ce dernier coagule. Une gamme de volume sanguin a été réalisée dans des tubes contenant 50 mL d'eau physiologique. Le traitement du sang a ensuite été réalisé comme décrit ci-dessus (voir figure 8A).

Les résultats présentés dans la figure 8B confirment que le polypeptide de SEQ n°1, sous forme de particules, est bien capable d'induire l'arrêt des saignements dans ce modèle que ce soit sous forme native ou concentrée. Ainsi, le volume moyen perdu avant l'arrêt d'un saignement en présence du polypeptide de SEQ n°1 est de 11.6 µL +/- 2.4 µL (non concentrée) et 12.6 µL +/- 6.1 µL (concentrée 10 fois) vs 42 µL +/- 18.4 µL pour les souris non traitées. La diminution de la perte sanguine est donc d'environ 70% par rapport aux souris témoins. A l'inverse, la perte de sang en présence d'héparine est fortement augmenté tel qu'attendu avec un volume moyen de sang perdu de 257.9 µL +/- 87.5. L'augmentation de la perte sanguine est donc de 514% par rapport aux souris témoins validant ainsi le modèle.

### REFERENCES

Spahn D et coll. Critical Care 2013, 17:R76
Fries D. Transfusion 2013; 53:91S-95S
Lashof-Sullivan M et coll. Nanoscale 2013, 5, 10719-10728
Modery-Pawlowski C et coll. Biomaterials 2013: 516-541
Clementson K. ThrombosisResearch 2012 129 220-224
Pires M et Chmielewski J. J. Am. Chem. Soc. 2009 131, 2706-2712
Przybyla D et coll. J. Am. Chem. Soc. 2013, 135, 3418-3422
Kojima C et coll. J. Am. Chem. Soc. 2009 131, 30652-6053
Slatter D et coll. Peptides 2012 36, 86-93
Kar et coll. Jounal of Biological Chemistry 2006 vol 281, n°44 33283-33290
Mazepa M et coll. ATVB 2013, 33(8) :1747-52
Bonhomme F et coll. Eur J Intern Med. 2014, 25(3):213-20
Beynon C et coll. Crit Care. 2012 Jul 26;16(4):228
Jenkins DH et coll. Shock. 2014 May;41 Suppl 1:3-12
Murdock AD et coll. Shock. 2014 May;41 Suppl 1:62-9
An B et coll. Frontiers in chemistry. 2014 June;2 article 40

## Revendications

1. Préparation injectable pour son utilisation dans le traitement des hémorragies comprenant :
- des particules obtenues par auto-assemblage des protéines ou peptides, lesdites particules ayant un diamètre moyen allant de 0.05 µm à 6 µm et lesdites particules comprenant des protéines ou peptides induisant l'adhésion et l'activation des *plaquettes* ; et
- au moins un véhicule ou excipient pharmaceutiquement acceptable, dans laquelle les protéines sont choisies parmi les collagènes ou les protéines recombinantes dont la séquence inclut au moins un polypeptide choisi parmi :
- le polypeptide de la séquence ID n°1;
- le polypeptide ayant la séquence de la position 25 à 184 de la séquence ID n°1 ;
- un polypeptide présentant au moins 70% d'identité sur toute sa longueur avec le polypeptide de la séquence ID n°1 ou avec le polypeptide ayant la séquence de la position 25 à 184 de la séquence ID n°1 ;
- un polypeptide ayant des séquences formées de la répétition de triplets GXY où G désigne la glycine et X et Y n'importe quels acides aminés répétés n fois avec n supérieur ou égal à 2, ou dans laquelle les peptides sont choisis parmi :
- le polypeptide de la séquence ID n°1;
- le polypeptide ayant la séquence de la position 25 à 184 de la séquence ID n°1 ;
- un polypeptide présentant au moins 70% d'identité sur toute sa longueur avec le polypeptide de la séquence ID n°1 ou avec le polypeptide ayant la séquence de la position 25 à 184 de la séquence ID n°1 ;
- un polypeptide ayant des séquences formées de la répétition de triplets GXY où G désigne la glycine et X et Y n'importe quels acides aminés répétés n fois avec n supérieur ou égal à 2.

2. Préparation injectable pour utilisation selon la revendication 1 dans laquelle les particules consistent en des peptides ou protéines choisies parmi les collagènes ou les protéines recombinantes.

3. Préparation injectable pour utilisation selon la revendication 2 dans laquelle les particules sont obtenues par coacervation, agrégation ou auto-assemblage.

4. Préparation injectable pour utilisation selon l'une des revendications précédentes dans laquelle le collagène ou les protéines sont pégylées ou pasylées.

5. Particules obtenues par auto-assemblage des protéines ou peptides, lesdites particules ayant un diamètre moyen allant de 0.05 µm à 6 µm ; lesdites particules comprenant des protéines ou peptides induisant l'adhésion et l'activation des plaquettes ; et **caractérisées en ce que** les protéines sont choisies parmi les collagènes ou les protéines recombinantes dont la séquence inclut au moins un polypeptide choisi parmi :
- le polypeptide de la séquence ID n°1;
- le polypeptide ayant la séquence de la position 25 à 184 de la séquence ID n°1 ;
- un polypeptide présentant au moins 70% d'identité sur toute sa longueur avec le polypeptide de la séquence ID n°1 ou avec le polypeptide ayant la séquence de la position 25 à 184 de la séquence ID n°1 ;
- un polypeptide ayant des séquences formées de la répétition de triplets GXY où G désigne la glycine et X et Y n'importe quels acides aminés répétés n fois avec n supérieur ou égal à 2,
ou **caractérisées en ce que** les peptides sont choisis parmi :
- le polypeptide de la séquence ID n°1;
- le polypeptide ayant la séquence de la position 25 à 184 de la séquence ID n°1 ;
- un polypeptide présentant au moins 70% d'identité sur toute sa longueur avec le polypeptide de la séquence ID n°1 ou avec le polypeptide ayant la séquence de la position 25 à 184 de la séquence ID n°1 ;
- un polypeptide ayant des séquences formées de la répétition de triplets GXY où G désigne la glycine et X et Y n'importe quels acides aminés répétés n fois avec n supérieur ou égal à 2.

## Patentansprüche

1. Injizierbare Zubereitung für deren Verwendung bei der Behandlung von Hämorrhagien, umfassend:
- Partikel, erhalten durch Selbstverbindung der Proteine oder Peptide, wobei die Partikel einen mittleren Durchmesser von 0,05 µm bis 6 µm haben und die Partikel Proteine oder Peptide umfassen, die die Adhäsion und die Aktivierung der Plättchen induzieren; und
- mindestens ein pharmazeutisch akzeptables Vehikel oder Hilfsstoff, wobei die Proteine aus den Kollagenen oder den rekombinanten Proteinen ausgewählt sind, deren Sequenz mindestens ein Polypeptid einschließt, ausgewählt aus:
- dem Polypeptid der Sequenz ID Nr. 1;
- dem Polypeptid, das die Sequenz der Position 25 bis 184 der Sequenz ID Nr. 1 hat;
- einem Polypeptid, das mindestens 70 % Identität über seine gesamte Länge mit dem Polypeptid der Sequenz ID Nr. 1 oder mit dem Polypeptid aufweist, das die Sequenz der Position 25 bis 184 der Sequenz ID Nr. 1 hat;
- einem Polypeptid, das Sequenzen hat, gebildet von der Wiederholung von Triplets GXY, wobei G Glycin und X und Y beliebige Aminosäuren in n-facher Wiederholung mit n größer oder gleich 2 bedeutet, oder wobei die Peptide ausgewählt sind aus:
- dem Polypeptid der Sequenz ID Nr. 1;
- dem Polypeptid, das die Sequenz der Position 25 bis 184 der Sequenz ID Nr. 1 hat;
- einem Polypeptid, das mindestens 70 % Identität über seine gesamte Länge mit dem Polypeptid der Sequenz ID Nr. 1 oder mit dem Polypeptid aufweist, das die Sequenz der Position 25 bis 184 der Sequenz ID Nr. 1 hat;
- einem Polypeptid, das Sequenzen hat, gebildet von der Wiederholung von Triplets GXY, wobei G Glycin und X und Y beliebige Aminosäuren in n-facher Wiederholung mit n größer oder gleich 2 bedeutet.

2. Injizierbare Zubereitung für Verwendung nach Anspruch 1, wobei die Partikel aus Peptiden oder Proteinen bestehen, die aus den Kollagenen oder den rekombinanten Proteinen ausgewählt sind.

3. Injizierbare Zubereitung für Verwendung nach Anspruch 2, wobei die Partikel durch Coacervation, Aggregation oder Selbstverbindung erhalten sind.

4. Injizierbare Zubereitung für Verwendung nach einem der vorangehenden Ansprüche, wobei das Kollagen oder die Proteine pegyliert oder pasyliert sind.

5. Partikel, erhalten durch Selbstverbindung der Proteine oder Peptide, wobei die Partikel einen mittleren Durchmesser von 0,05 µm bis 6 µm haben;
wobei die Partikel Proteine oder Peptide umfassen, die die Adhäsion und die Aktivierung der Plättchen induzieren; und
**dadurch gekennzeichnet, dass** die Proteine aus den Kollagenen oder den rekombinanten Proteinen ausgewählt sind, deren Sequenz mindestens ein Polypeptid einschließt, ausgewählt aus:
- dem Polypeptid der Sequenz ID Nr. 1;
- dem Polypeptid, das die Sequenz der Position 25 bis 184 der Sequenz ID Nr. 1 hat;
- einem Polypeptid, das mindestens 70 % Identität über seine gesamte Länge mit dem Polypeptid der Sequenz ID Nr. 1 oder mit dem Polypeptid aufweist, das die Sequenz der Position 25 bis 184 der Sequenz ID Nr. 1 hat;
- einem Polypeptid, das Sequenzen hat, gebildet von der Wiederholung von Triplets GXY, wobei G Glycin und X und Y beliebige Aminosäuren in n-facher Wiederholung mit n größer oder gleich 2 bedeutet,
oder **gekennzeichnet dadurch, dass** die Peptide ausgewählt sind aus:
- dem Polypeptid der Sequenz ID Nr. 1;
- dem Polypeptid, das die Sequenz der Position 25 bis 184 der Sequenz ID Nr. 1 hat;
- einem Polypeptid, das mindestens 70 % Identität über seine gesamte Länge mit dem Polypeptid der Sequenz ID Nr. 1 oder mit dem Polypeptid aufweist, das die Sequenz der Position 25 bis 184 der Sequenz ID Nr. 1 hat;
- einem Polypeptid, das Sequenzen hat, gebildet von der Wiederholung von Triplets GXY, wobei G Glycin und X und Y beliebige Aminosäuren in n-facher Wiederholung mit n größer oder gleich 2 bedeutet.

## Claims

1. An injectable preparation for use in the treatment of hemorrhages comprising:
- particles obtained by self-assembly of proteins or peptides, said particles having a mean diameter ranging from 0.05 µm to 6 µm and said particles comprising proteins or peptides inducing the adhesion and activation of platelets; and
- at least one pharmaceutically acceptable vehicle or excipient, wherein the proteins are chosen from among collagens or recombinant proteins whose sequence includes at least one polypeptide chosen from among:
- the polypeptide of sequence ID No. 1;
- the polypeptide having the sequence from position 25 to 184 of sequence ID No. 1;
- a polypeptide having at least 70% identity along its entire length with the polypeptide of sequence ID No. 1 or with the polypeptide having the sequence from position 25 to 184 of sequence ID No. 1;
- a polypeptide having sequences formed from the repetition of GXY triplets, where G designates glycine and X and Y designate any amino acid, repeated n times with n greater than or equal to 2, or wherein the peptides are chosen from among:
- the polypeptide of sequence ID No. 1;
- the polypeptide having the sequence from position 25 to 184 of sequence ID No. 1;
- a polypeptide having at least 70% identity along its entire length with the polypeptide of sequence ID No. 1 or with the polypeptide having the sequence from position 25 to 184 of sequence ID No. 1;
- a polypeptide having sequences formed from the repetition of GXY triplets, where G designates glycine and X and Y designate any amino acid, repeated n times with n greater than or equal to 2.

2. The injectable preparation for use according to claim 1 wherein the particles consist of peptides or proteins chosen from among collagens or recombinant proteins.

3. The injectable preparation for use according to claim 2 wherein the particles are obtained by coacervation, aggregation or self-assembly.

4. The injectable preparation for use according to one of the preceding claims wherein the collagen or proteins are pegylated or pasylated.

5. Particles obtained by self-assembly of proteins or peptides, said particles having a mean diameter ranging from 0.05 µm to 6 µm;
said particles comprising proteins or peptides inducing adhesion and activation of platelets; and
**characterized in that** the proteins are chosen from among collagens or recombinant proteins whose sequence includes at least one polypeptide chosen from among:
- the polypeptide of sequence ID No. 1;
- the polypeptide having the sequence from position 25 to 184 of sequence ID No. 1;
- a polypeptide having at least 70% identity along its entire length with the polypeptide of sequence ID No. 1 or with the polypeptide having the sequence from position 25 to 184 of sequence ID No. 1;
- a polypeptide having sequences formed from the repetition of GXY triplets, where G designates glycine and X and Y designate any amino acid, repeated n times with n greater than or equal to 2,
or **characterized in that** the peptides are chosen from among:
- the polypeptide of sequence ID No. 1;
- the polypeptide having the sequence from position 25 to 184 of sequence ID No. 1;
- a polypeptide having at least 70% identity along its entire length with the polypeptide of sequence ID No. 1 or with the polypeptide having the sequence from position 25 to 184 of sequence ID No. 1;
- a polypeptide having sequences formed from the repetition of GXY triplets, where G designates glycine and X and Y designate any amino acid, repeated n times with n greater than or equal to 2.


### SEQUENCE LISTING

<110> NVH Médicinal
   David VANDROUX
   CENTRE HOSPITALIER UNIVERSITAIRE DE DIJON
<120> PREPARATIONS INJECTABLES A BASE DE COLLAGENES CAPABLES DE CONTROLER LES SAIGNEMENTS ET/OU DE SE SUBSTITUER A DES PLAQUETTES DANS LE CAS DE SYNDROMES HEMORRAGIQUES
<130> D33216
<150> FR 1362418
   <151> 2013-12-11
<160> 1
<170> PatentIn version 3.5
<210> 1
   <211> 184
   <212> PRT
   <213> Artificial Sequence
<220>
   <223> Synthetic Construct
   <400> 1
   Met Met Ser Phe Val Gln Lys Gly Ser Trp Leu Leu Leu Ala Leu Leu
   1               5                   10                  15
   His Pro Thr Ile Ile Leu Ala Gln Gly Arg Pro Gly Ala Pro Gly Glu
               20                  25                  30
   Arg Gly Leu Pro Gly Pro Pro Gly Pro Arg Gly Ala Ala Gly Glu Pro
           35                  40                  45
   Gly Arg Asp Gly Val Pro Gly Gly Pro Gly Met Arg Gly Met Pro Gly
       50                  55                  60
   Ser Pro Gly Gly Pro Gly Ser Asp Gly Lys Pro Gly Pro Pro Gly Ser
   65                  70                  75                  80
   Gln Gly Glu Ser Gly Arg Pro Gly Pro Pro Gly Glu Asn Gly Phe Pro
                   85                  90                  95
   Gly Glu Arg Gly Asp Ala Gly Ala Pro Gly Ala Pro Gly Pro Arg Gly
               100                 105                 110
   Gln Pro Gly Val Met Gly Phe Pro Gly Asp Ala Gly Ala Pro Gly Ala
           115                 120                 125
   Pro Gly Pro Arg Gly Gln Pro Gly Val Met Gly Phe Pro Gly Pro Pro
       130                 135                 140
   Gly Pro Pro Gly Pro Pro Gly Pro Pro Gly Pro Pro Gly Pro Pro Gly
   145                 150                 155                 160
   Pro Pro Gly Pro Pro Gly Pro Pro Gly Pro Pro Gly Pro Arg Gly Asp
                   165                 170                 175
   Lys Gly Pro Pro Gly Pro Gly Ser
               180